# EUROPÄISCHE PATENTSCHRIFT

(11) **EP 1 340 482 B1**
(45) Veröffentlichungstag und Bekanntmachung des Hinweises auf die Patenterteilung: **30.11.2005**
(21) Anmeldenummer: 03450052.0
(22) Anmeldetag: 26.02.2003
(51) Int. Cl.: A61K 6/04

(54) **Dentallegierung auf Edelmetallbasis**
Precious metal-based dental alloy
Alliage dentaire à base de métaux précieux

(30) Priorität: 27.02.2002 AT 20020305
(43) Veröffentlichungstag der Anmeldung: 03.09.2003
(73) Patentinhaber: Elsa, Edelmetall Legier-und Scheideanstalt GMBH, A-2103 Langenzersdorf (AT)
(72) Erfinder: Esterle, Astrid, A-1220 Wien (AT)
(74) Vertreter: Pfeifer, Otto

(56) Entgegenhaltungen:
- DE-A- 2 440 425
- DE-A- 3 830 666
- DE-C- 373 725
- DE-C- 3 819 904
- NL-A- 9 200 566
- US-A- 4 389 370
- US-A- 5 462 437

## Beschreibung

Die vorliegende Erfindung betrifft eine neue Dentallegierung auf Edelmetallbasis, welche unter Erfüllung der in den entsprechenden Normen geforderten Eigenschaften hinsichtlich mechanischer Festigkeit, Korrosionsbeständigkeit, Verarbeitbarkeit im Dentallabor usw. einen besonders niedrigen Goldgehalt aufweist.

Anwendungsgebiete für die neue Dentallegierung sind Kronen, Brücken, Inlays und Prothetikarbeiten.

Die neue Legierung kann in dentaltechnisch üblicher Weise keramisch verblendet werden.

Auf dem Markt haben sich Legierungen auf Palladiumbasis, auch solche mit hohen Silbergehalten, seit Jahren bewährt, sie werden aber aus Gründen der aktuellen Marktsituation immer weniger appliziert. Auch gab es Schwierigkeiten in Bezug auf die Verträglichkeit dieser bekannten Legierungen auf Palladiumbasis, die auf ungewollte, nicht spezifizierte Kupferverunreinigungen, die in derartigen Produkten in verhältnismäßig hoher Menge vorlagen, zurückzuführen waren.

Aufgabe der vorliegenden Erfindung war es, eine Dentallegierung auf Goldbasis mit hohem Silbergehalt und besonders niedrigem Palladiumgehalt zu schaffen, die biokompatibel ist und für niedrigschmelzende Keramiken geeignet ist.

Diese Aufgaben werden mit der in den Ansprüchen gekennzeichneten silberreichen, palladiumarmen Dentallegierung auf Goldbasis gelöst.

Infolge der Verwendung höchstreiner Metalle als Ausgangsstoffe bereiten die erfindungsgemäßen Legierungen keine Schwierigkeiten in Bezug auf Verträglichkeit.

Bisher am Markt erhältliche silberreiche Legierungen sind primär für niedrigschmelzende Keramiken geeignet, enthalten kein Gold (was korrosionstechnisch ein bewährter und patientenfreundlicher Legierungsbestandteil wäre), hingegen aber 40% Palladium, dessen Allergenität nicht ganz geklärt erscheint, die aber - wie erfindungsgemäß vorgesehen - durch die gleichzeitige Anwendung von Gold jedenfalls deutlich reduziert ist. Derartige Legierungen vom PMNF-Typ sind nach wie vor invasiv verbreitet im Einsatz und verursachen keine gesundheitlichen Probleme.

Probleme gab es hingegen in den vergangenen Jahren mit den Keramiküberzügen. Um eine dauerhafte Verwendung im Munde des Patienten zu ermöglichen, ist verarbeitungstechnisch eine entsprechende Sorgfalt seitens der Dentaltechnik zu fordern, und zwar sowohl hinsichtlich Verunreinigungen als auch bezüglich der Einhaltung des angegebenen Schmelzbereiches. Darüber hinaus sind heutzutage Keramikmassen verfügbar, die infolge des Silbergehaltes in der Dentallegierung nicht mehr dunkler werden, wodurch auch die dem Stand der Technik vor zehn bis zwanzig Jahren entsprechenden Probleme nunmehr als überwunden angesehen werden können.

Der fakultative Einsatz von Platin in den erfindungsgemäßen Dentallegierungen ermöglicht neben der bereits angesprochenen Erhöhung der Schmelztemperatur, was für hochschmelzende Keramikmassen wesentlich ist, auch eine bessere Korrosionsbeständigkeit im Vergleich zu früheren Silberbasislegierungen.

Ein erheblicher Vorteil der erfindungsgemäßen Dentallegierungen gegenüber den bekannten nicht aufbrennfähigen Silberlegierungen liegt darin, daß sie aufbrennfähig sind.

Die Erfindung wird durch die nachfolgenden Beispiele näher erläutert.

### Beispiel 1: Aufbrennfähige Silberlegierung

### Legierungszusammensetzung:

| | |
|---|---|
| Au | 24,5% |
| Pd | 20,0% |
| Ru | 0,5% |
| Ag | 47,0% |
| Zn | 4,0% |
| Sn | 3,0% |
| In | 1,0% |

| | |
|---|---|
| Vickershärte HV₅ (nach Guß) | 200 |
| Schmelzintervall | 965-1040°C |
| WAK | 17,0 |
| 20-600 | |

### Beispiel 2: Aufbrennfähige Silberlegierung

### Legierungszusammensetzung:

| | |
|---|---|
| Au | 22,0% |
| Pt | 4,0% |
| Pd | 20,0% |
| Ru | 1,0% |
| Ag | 47,0% |
| Zn | 4,0% |
| Sn | 1,0% |
| In | 1,0% |

| | |
|---|---|
| Vickershärte HV₅ (nach Guß) | 210 |
| Schmelzintervall | 1010-1110°C |
| WAK | 16,7 |
| 20-600 | |

### Beispiel 3: Aufbrennfähige Silberlegierung

### Legierungszusammensetzung:

| | |
|---|---|
| Au | 26,0% |
| Pt | 10,0% |
| Pd | 25,0% |
| Ag | 33,0% |
| Zn | 4,0% |
| Ru | 1,0% |
| In | 1,0% |

### Vergleichsbeispiel: Nicht aufbrennfähige Silberlegierung Legierungszusammensetzung

| | |
|---|---|
| Au | 20,0% |
| Pd | 20,0% |
| Ag | 40,0% |
| Zn | 2,0% |
| In | 18,0% |
| Ir | 0,1% |

| | |
|---|---|
| Vickershärte HV₅ | 150 |
| Schmelzintervall | 880-925°C |
| Löslichkeit (µg/cm² x 7d) | <100 |

## Patentansprüche

1. Dentallegierung auf Edelmetallbasis, **gekennzeichnet durch** die Konzentrationsbereiche der folgenden Elemente:
| | |
|---|---|
| Palladium | 18,0% bis 42% |
| Silber | 33,0% bis 48% |
| Gold | 10,0% bis 30% |
| Ruthenium | 0,1% bis 2% |
| Zink | 2,0% bis 6% |
| Indium | 1,0% bis 4% |
und **durch** einen zusätzlichen Gehalt an den folgenden Elementen:
| | |
|---|---|
| Platin | 0% bis 10% |
| Rhodium | 0% bis 5% |
| Tantal | 0% bis 2% |
| Wolfram | 0% bis 3% |
| Molybdän | 0% bis 2% |
| Rhenium | 0% bis 1% |
| Gallium | 0% bis 6% |
| Germanium | 0% bis 1% |
| Nio | 0% bis 3% |
| Zirkonium | 0% bis 1% |
| Zinn | 0% bis 4% |
| Sonstige Nichtedelmetalle | insgesamt bis zu 1% (wie z.B. Fe, Mn, Cr, Co, Ti) |
| Sonstige Edelmetalle | (wie Ir, Os) insgesamt bis zu 1% |
und **durch** ein Freisein von Nickel und von Kupfer.

2. Dentallegierung nach Anspruch 1, **dadurch gekennzeichnet, daß** sie frei von Gallium ist.

3. Verwendung der Dentallegierung nach Anspruch 1 oder 2 zum Verblenden mit Dentalkeramikmassen.

## Claims

1. Dental alloy based on noble metal, **characterised by** the concentration ranges of the following elements:
| | |
|---|---|
| palladium | 18.0% to 42% |
| silver | 33.0% to 48% |
| gold | 10.0% to 30% |
| ruthenium | 0.1 % to 2% |
| zinc | 2.0% to 6% |
| indium | 1.0% to 4% |
and by an additional content of the following elements:
| | |
|---|---|
| platinum | 0% to 10% |
| rhodium | 0% to 5% |
| tantalum | 0% to 2% |
| tungsten | 0% to 3% |
| molybdenum | 0% to 2% |
| rhenium | 0% to 1% |
| gallium | 0% to 6% |
| germanium | 0% to 1% |
| niobium | 0% to 3% |
| zirconium | 0% to 1% |
| tin | 0% to 4% |
| other non-noble metals | in total up to 1% (such as for example Fe, Mn, Cr, Co, Ti) |
| other noble metals | (such as Ir, Os) in total up to 1% |
and by an absence of nickel and of copper.

2. Dental alloy according to claim 1, **characterised in that** it is free of gallium.

3. Use of the dental alloy according to claim 1 or 2 for veneering with dental ceramic compositions.

## Revendications

1. Alliage dentaire à base de métaux nobles, **caractérisé par** les gammes de concentration des éléments suivants :
| | |
|---|---|
| palladium | 18,0% à 42% |
| argent | 33,0% à 48% |
| or | 10,0% à 30% |
| ruthénium | 0,1% à 2% |
| zinc | 2,0% à 6% |
| indium | 1,0% à 4% |
et par une teneur supplémentaire en éléments suivants :
| | |
|---|---|
| platine | 0% à 10% |
| rhodium | 0% à 5% |
| tantale | 0% à 2% |
| tungstène | 0% à 3% |
| molybdène | 0% à 2% |
| rhénium | 0% à 1% |
| gallium | 0% à 6% |
| germanium | 0% à 1% |
| niobium | 0% à 3% |
| zirconium | 0% à 1% |
| étain | 0% à 4% |
| autres métaux communs | au total jusqu'à 1% (par exemple Fe, Mn, Cr, Co, Ti) |
| autres métaux nobles | (par exemple Ir, Os) au total jusqu'à 1% |
et par une absence de nickel et de cuivre.

2. Alliage dentaire selon la revendication 1, **caractérisé en ce qu'**il est exempt de gallium.

3. Utilisation de l'alliage dentaire selon la revendication 1 ou 2 à revêtir avec des céramiques dentaires.
